# EUROPEAN PATENT SPECIFICATION

(11) **EP 2 608 836 B1**
(45) Date of publication and mention of the grant of the patent: **28.03.2018**
(21) Application number: 11764614.1
(22) Date of filing: 25.07.2011
(51) Int. Cl.: A61M 16/16

(54) **PORTABLE HUMIDIFICATION SYSTEM AND ADAPTOR THEREFORE**
TRAGBARES BEFEUCHTUNGSSYSTEM UND ADAPTER DAFÜR
SYSTÈME D'HUMIDIFICATION PORTABLE ET ADAPTATEUR CORRESPONDANT

(30) Priority: 27.08.2010 US 377472 P
(43) Date of publication of application: 03.07.2013
(73) Proprietor: Koninklijke Philips N.V., 5656 AE Eindhoven (NL)
(72) Inventor: HO, Peter, Chi, Fai, NL-5656 AE Eindhoven (NL)
(74) Representative: van Velzen, Maaike Mathilde
(86) International application number: PCT/IB2011/053293
(87) International publication number: WO 2012/025846

(56) References cited:
- EP-A1- 0 140 838
- WO-A1-2009/015410
- GB-A- 2 072 526
- US-A- 3 944 635
- US-A- 3 990 441
- US-A- 4 011 288
- US-A- 4 039 639
- US-A- 4 132 883
- US-A- 4 225 542
- US-A- 4 366 105
- US-A- 5 916 493

## Description

### BACKGROUND OF THE INVENTION

### 1. Field of the Invention

The present invention relates to airway pressure support systems, and, more particularly, to a portable airway pressure support system having a humidification system. The invention further relates to humidification systems and adaptors therefor.

### 2. Description of the Related Art

There are numerous situations where it is necessary or desirable to deliver a pressurized flow of breathing gas non-invasively to the airway of a patient, i.e., without intubating the patient or surgically inserting a tracheal tube in the esophagus. For example, it is known to ventilate a patient using a technique known as non-invasive ventilation. It is also known to deliver continuous positive airway pressure (CPAP) or variable airway pressure, such as a bi-level pressure that varies with the patient's respiratory cycle or an auto-titrating pressure that varies with the monitored condition of the patient. Typical pressure support therapies are provided to treat a medical disorder, such as sleep apnea syndrome, in particular, obstructive sleep apnea (OSA), or congestive heart failure.

During such treatments, a supply of pressurized gas is typically supplied to a patient through a patient interface, such as a nasal, oral, or combination nasal/oral mask. The continuous flow of air from such PAP devices can be irritating to the tissues of the nose, mouth, and/or throat. This irritation may result in nosebleeds, increased mucous, congestion, and coughing or sneezing. The main reason for such irritation is the drying effect of the pressurized gas.

To help alleviate such effects, humidifiers are frequently provided between or integral with a PAP machine and the user interface in order to humidify the
otherwise relatively-dry compressed air generated by the PAP machine. Typically, humidifiers can be categorized as passover types or non-passover types. In a passover type of humidifier, water is contained in a reservoir that may or may not be heated. While the water is allowed to evaporate to produce vapor within the reservoir, breathing gas is passed over the surface of the water. Increased water vapor within the reservoir increases the capability to provide more humidity to the gas that is delivered to a user. In a non-passover type of humidifier, water is delivered into the gas stream via nebulization, atomization, vaporization, or a combination thereof. Independent of the type of humidification used, in current positive airway pressure support systems the control of humidification is such that the output of humidity is relatively constant or on/off based on the user's demand.

Such humidifiers are generally of a size that is inconvenient for travel use. Further, even those humidifiers that are sized in a manner for potential travel use are accompanied by the inconvenience of discharging leftover water and drying the units prior to packing. Such inconvenience often deters patients from using such units while traveling.

US 3,990,441 A, WO 2009/015410 A1, GB 2072526 A, US 4,366, 105 A and US 4,225,542 A disclose various embodiments of a humidifier for use in an oxygen delivery system for delivering oxygen to a patient, which all require the use of a special container for holding a humidifying fluid.

US 5,916,493 discloses a humidifier system for producing a humidified and heated breathing gas including a humidifier module having a humidifying chamber with a supply gas inlet, a breathing gas outlet and a heat transfer element. A tube extending between a liquid reservoir and the humidifier module includes therein an elongate liquid absorbing wick whose upper end has a plurality of leaves extending planarly and radially outwardly therefrom into the humidifying chamber into contact with said heat transfer element. Liquid is transported by the wick upwardly into the leaves thereof where it is evaporated and mixed with the supply gas in the humidifying chamber to generate a heated and humidified breathing gas. A vent passage between the humidifying chamber and the liquid reservoir allows the automatic adjustment of the humidifying chamber and the liquid reservoir allows the automatic adjustment of the pressure in the reservoir and thus the capillary flow of liquid up the wick in response to a change in the volumetric rate of supply gas so as to provide a humidifier system where the heat and humidity level of the breathing gas are self-regulated. US 4,039,639 A, US 3,944,635 A and US 4,011,288 A discloses the use of bottles for holding a humidifier liquid.

### SUMMARY OF THE INVENTION

Accordingly, it is an object of the present invention to provide an interface system that overcomes the shortcomings of conventional humidification systems. This object is achieved according to one embodiment of the present invention as defined in claim 1 by providing an adaptor for use in a humidification system for humidifying a flow of pressurized gas to a patient interface device is provided. The adaptor comprises: an inlet structured to be coupled to a supply of pressurized gas, an outlet structured to be coupled to a patient interface device, a port having female threads structured to be coupled to a male threaded opening of a bottle, a first passage extending between the inlet and the port, a second passage extending between the outlet and the port, and a partition disposed generally between the first passage and the second passage proximate the port. The partition is structured to effectively separate the port and the threaded opening of the bottle into two portions, when the adaptor is coupled to the bottle. The first passage is structured to direct a flow of pressurized gas from the inlet to the first portion of the port and the second passage is structured to direct a flow of gas from the second portion of the port to the outlet. The partition comprises an aperture disposed therein, the aperture housing a heating element. One or more of the first and second passages comprises a sensor disposed therein for detecting the temperature of the pressurized gas.

According to another embodiment of the present invention, a system for humidifying a flow of pressurized gas to a patient interface device is provided. The system comprises: an adaptor according to the present invention and a bottle.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1 is a schematic diagram of pressure support system according to one particular, non-limiting embodiment in which the present invention in its various embodiments may be implemented;
FIG. 2A is a schematic cross-sectional view of a humidification system;
FIG. 2B is a schematic view of a portion of the humidification system of FIG. 2A taken along B-B of FIG. 2A.
FIG. 3A is a schematic cross-sectional view of a humidification system according to a non-limiting embodiment of the present invention employing the adaptor of FIG. 2;
FIG. 3B is a schematic view of a portion of the humidification system of FIG. 3A taken along B-B of FIG. 3A; and
FIG. 4 is a schematic cross-sectional view of a humidification system.

### DETAILED DESCRIPTION OF THE EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

The present invention addresses shortcomings of the prior art by providing a humidification system that utilizes an adaptor that can be readily mated to a disposable bottle, such as an inexpensive polyethylene bottle that is commonly sold containing soda, water, or other liquid. By utilizing such a readily available disposable bottle, a travelling user (hereinafter referred to as a "patient") of a humidification system in accordance with the present invention does not need to pack a bulky storage tank, such as commonly associated with known humidification systems. Instead, a travelling patient merely needs to pack a compact adaptor that can be used with an inexpensive, disposable plastic bottle which can be readily sourced at the travelling patient's destination. After use, the disposable bottle can then be simply discarded, thus eliminating any need to clean or dry after use for subsequent use or packing.

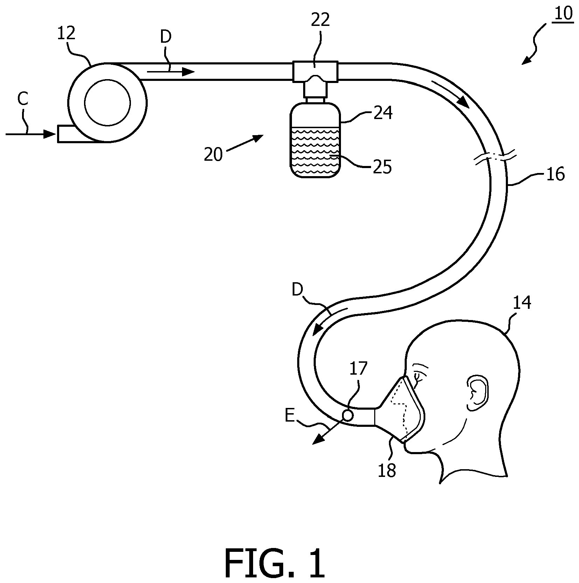

FIG. 1 is a schematic diagram of a pressure support system 10 according to one particular, non-limiting embodiment in which the present invention in its various embodiments may be implemented. Referring to FIG. 1, pressure support system 10 includes a gas flow generator 12, such as a blower used in a conventional CPAP or bi-level pressure support device, which receives breathing gas, generally indicated by arrow C, from any suitable source, e.g., a pressurized tank of oxygen or air, the ambient atmosphere, or a combination thereof. Gas flow generator 12 generates a flow of breathing gas, such as air, oxygen, or a mixture thereof, for delivery to an airway of a patient 14 at relatively higher and lower pressures, i.e., generally equal to or above ambient atmospheric pressure. In the exemplary embodiment, gas flow generator 12 is capable of providing a flow of breathing gas generally ranging in pressure from 3-30 cmH₂O.

The pressurized flow of breathing gas, generally indicated by arrow D, from gas flow generator 12 is delivered via a delivery conduit 16 to a patient interface 18. Patient interface 18 can be of any known construction, such as a nasal mask, nasal/oral mask, nasal cannula, total face mask, tracheal tube, endotracheal tube, or any of the device that is typically worn by, or otherwise attached to, patient 14 to communicate the flow of breathing gas to the airway of patient. Delivery conduit 16 is also typically referred to as a patient circuit. For present purposes, an interface system is defined as the combination of a patient interface and at least a portion of conduit 16.

Pressure support system 10 of FIG. 1 is what is known as a single-limb system, meaning that the patient circuit includes only delivery conduit 16 connecting patient 14 to pressure support system 10. As such, an exhaust vent 17 is provided in delivery conduit 16 for venting exhaled gasses from the system as indicated by arrow E. It should be noted that exhaust vent 17 can be provided at other locations in addition to, or instead of, in delivery conduit 16, such as in patient interface 18. It should also be understood that exhaust vent 17 can have a wide variety of configurations depending on the desired manner in which gas is to be vented from pressure support system 10. The present invention can be used in either a single-limb or dual limb system.

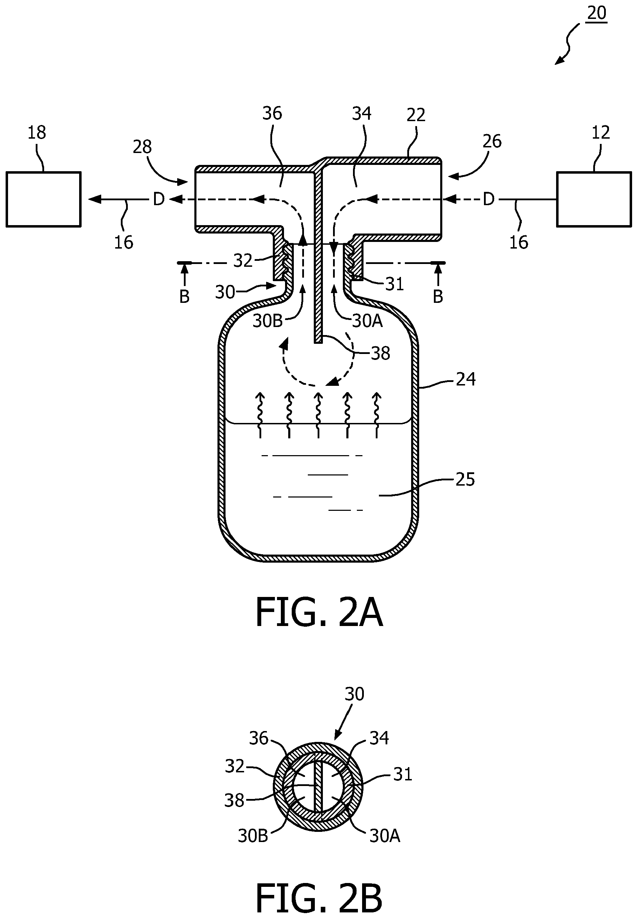

Continuing to refer to FIG. 1, pressure support system 10 further includes a humidification system 20 disposed along delivery conduit 16 at a location between gas flow generator 12 and patient interface device 18. FIGS. 2A and 2B show an example humidification system 20.

Humidification system 20 includes an adaptor 22 and a container, such as bottle 24, that is adapted to house a volume of liquid 25 (e.g., without limitation, water, scented water, tap water, distilled water, spring water) for humidifying flow D of pressurized gas. In an exemplary embodiment, adaptor 22 is formed from plastic or other suitable material (e.g., without limitation, light metal such as aluminum) and includes an inlet 26, an outlet 28 and a port 30. Inlet 26 is structured to be coupled to a supply of pressurized gas, such as gas flow D from flow generator 12, and outlet 28 is structured to be coupled to a patient interface, such as patient interface 18. Such couplings at inlet 26 and outlet 28 is typically accomplished through use of a conduit, such as shown in pressure support system 10 previously discussed in regard to FIG. 1. Accordingly, inlet 26 and outlet 28 may be provided with suitable fitting to accommodate such coupling, for example, the adaptor may have rigid conical fittings made to follow the standards of common CPAP or mask system with ISO fittings, or may have flexible fittings (e.g., cuffs made out of rubber to fit the associated system).

As shown in FIG. 2B, port 30 is generally circular in shape and is structured to be coupled to a threaded opening 31 of bottle 24. To provide for such coupling, port 30 is provided with a threaded portion 32 that is structured to cooperatively engage threaded opening 31 of bottle 24.

Referring to FIG. 2A, adaptor 22 further includes a first passage 34 and a separate second passage 36 formed therein. First passage 34 generally extends between inlet 26 and port 30. Second passage 36 generally extends between port 30 and outlet 28. First passage 34 and second passage 36 are generally separated by a partition 38 which is disposed in adaptor 22 generally proximate port 30. When adaptor 22 is coupled to bottle 24, partition 38 effectively separates port 30 and threaded opening 31 of bottle 24 into two portions 30A, 30B, a first portion 30A being a portion of first passage 34, and the second portion 30B being a portion of second passage 36. Accordingly, it is to be appreciated that partition 38 separates first passage 34 from second passage 36. As will be discussed further below, first passage 34 is structured to direct a flow of pressurized gas received at inlet 26 from inlet 26 to port 30, and thus into bottle 24. Similarly, second passage 36 is structured to direct a flow of pressurized gas from port 30, received from bottle 24, to outlet 28.

Having thus described the basic structure of humidification system 20, its use will now be described. As shown by the dashed arrows in FIG. 2A, pressurized gas flow D, supplied (for example) by flow generator 12, enters first passage 34 of adaptor 22 via inlet 26. Flow D then exits first passage 34, and thus adaptor 22, via port 32. Upon exiting port 30 flow D enters bottle 24 where its vapor content is increased due to the presence of liquid 25 contained in bottle 24. Due to the continued pressure of new flow D entering bottle 24 via first passage 34, the portion of flow D already in bottle 24 is forced from bottle 24 via port 30 and into second passage 36. Such flow D then exits adaptor 22 via outlet 28 where it continues to patient interface 18.

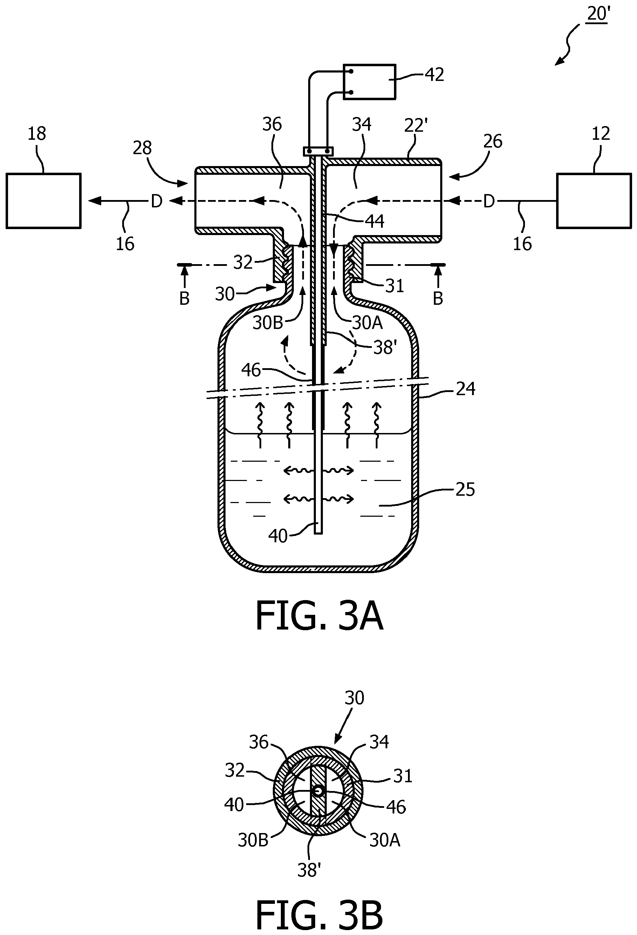

FIGS. 3A and 3B show an example of humidification system 20' in accordance with a non-limiting embodiment of the present invention. Humidification system 20' is of generally similar construction and function as humidification system 20, previously discussed. However, unlike system 20, humidification system 20' utilizes a heating element 40 powered by power supply 42 to heat liquid 25 contained in bottle 24 in order to enhance the transfer of liquid 25, and thus the humidity of flow D. In an exemplary embodiment, heating element 40 is formed from a highly conductive material (e.g., aluminum or copper) and is electrically coupled to power supply 42. Power supply 42 may be a DC power supply, however other suitable power supplies may be employed without varying from the scope of the present invention.

In order to accommodate heating element 40, an aperture 44, of appropriate dimensions to generally house heating element 40 therein, is provided in partition 38' of adaptor 22'. Heating element 40 may be generally surrounded by one or more insulating materials 46.

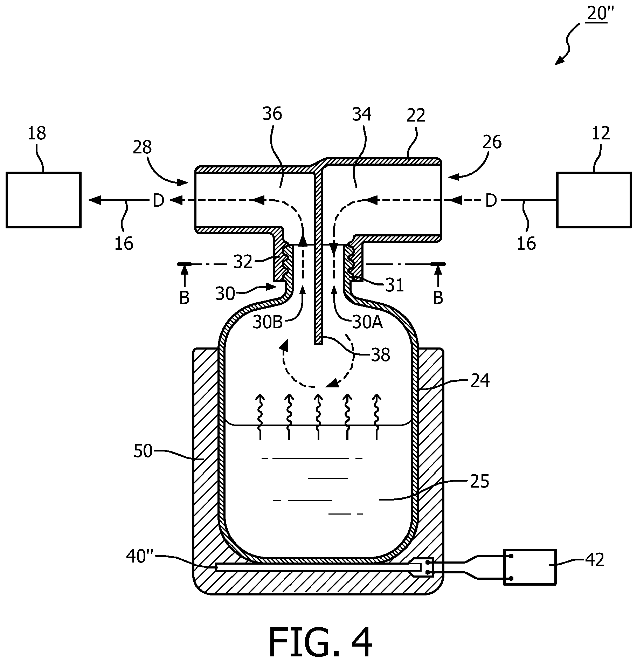

FIG. 4 shows an example of a humidification system 20". Humidification system 20" includes adaptor 22 and bottle 24 as previously described in connection with FIGS. 2A and B. Humidification system 20" further includes an outer member 50 that may be structured to enclose a portion of bottle 24. Outer member 50 may be formed from an insulative material that, as shown in FIG. 4, generally encloses a large portion of bottle 24. In an exemplary embodiment, such outer member 50 is utilized in conjunction with the placement of hot water 25 in bottle 24 by a patient. In such instance, outer member 50 would generally serve to keep hot water 25 at an elevated temperature for an extended period of time, thus providing for grater humidification of flow D passing through bottle 24. As further shown in FIG. 4, one or more heating elements 40" may also be provided in outer member 50 to heat liquid 25 contained in bottle 24. Heating element 40" can be provided at other locations in outer member 50.

Although not specifically shown in the depicted embodiments, it is to be appreciated that any of the adaptors described herein may be provided with one or more sensing devices (sensors) disposed along one or both of the first and second passages to monitor the flow of pressurized gas through all, or part, of the adaptor. Such one or more sensors may include sensors for detecting properties of the pressurized gas (e.g., without limitation, temperature, humidity, flow rate, pressure). Such sensors can communicate with the gas flow generator or other device via hardwire or wireless communication.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. An adaptor (22') for use in a humidification system (10) for humidifying a flow of pressurized gas to a patient interface device, the adaptor comprising:
an inlet (26) structured to be coupled to a supply of pressurized gas;
an outlet (28) structured to be coupled to a patient interface device;
a port (30) having female threads structured to be coupled to a male threaded opening (31) of a bottle;
a first passage (34) extending between the inlet and the port;
a second passage (36) extending between the outlet and the port; and
a partition (38') disposed generally between the first passage and the second passage proximate the port, wherein the partition is structured to effectively separate the port (30) and the threaded opening (31) of the bottle into two portions (30A, 30B), when the adaptor is coupled to the bottle, and wherein the first passage (34) is structured to direct a flow of pressurized gas from the inlet to the first portion (30A) of the port and wherein the second passage (36) is structured to direct a flow of gas from the second portion (30B) of the port to the outlet, **characterized in that** the partition comprises an aperture (44) disposed therein, the aperture housing a heating element (40), wherein one or more of the first and second passages comprise a sensor disposed therein for detecting the temperature of the pressurized gas.

2. The adaptor of claim 1, wherein the sensor is structured to detect one or more of the following properties of the pressurized gas: -humidity, flow rate, or pressure.

3. A system (10) for humidifying a flow of pressurized gas to a patient interface device, the system comprising
(a) an adaptor (22') as claimed in any one of claims 1 to 2; and
(b) a bottle (24) coupled to the port, the bottle being structured to house a quantity of liquid.

4. The system of claim 3, wherein the heating element disposed in the aperture and extends a distance into the bottle, a portion of the heating element being structured to engage the quantity of liquid.

5. The system of claim 4, wherein the heating element comprises a conductive material.

6. The system of claim 3, wherein the bottle comprises an outer member generally disposed about at least a portion of the bottle.

7. The system of claim 6, wherein the outer member comprises insulative materials.

8. The system of claim 6, wherein the outer member comprises a heating element.

## Patentansprüche

1. Adapter (22') zur Verwendung in einem Befeuchtungssystem (10) zum Befeuchten eines Druckgasstroms für eine Patientenschnittstellenvorrichtung, wobei der Adapter Folgendes umfasst:
einen Einlass (26), der strukturiert ist, um mit einer Druckgasversorgung gekoppelt zu sein;
einen Auslass (28), der strukturiert ist, um mit einer Patientenschnittstellenvorrichtung gekoppelt zu sein;
einen Stutzen (30), der ein Innengewinde aufweist, das strukturiert ist, um mit einer Öffnung (31) mit Außengewinde einer Flasche gekoppelt zu werden;
einen ersten Durchgang (34), der sich zwischen dem Einlass und dem Stutzen erstreckt;
einen zweiten Durchgang (36), der sich zwischen dem Auslass und dem Stutzen erstreckt; und
eine Trennwand (38'), die im Allgemeinen zwischen dem ersten Durchgang und dem zweiten Durchgang in der Nähe des Stutzens angeordnet ist, wobei die Trennwand strukturiert ist, um den Stutzen (30) und die Gewindeöffnung (31) der Flasche effektiv in zwei Abschnitte (30A, 30B) zu trennen, wenn der Adapter mit der Flasche gekoppelt ist, und wobei der erste Durchgang (34) strukturiert ist, um einen Druckgasstrom von dem Einlass zu dem ersten Abschnitt (30A) des Stutzens zu leiten, und wobei der zweite Durchgang (36) strukturiert ist, um einen Gasstrom von dem zweiten Abschnitt (30B) des Stutzens zu dem Auslass zu leiten, **dadurch gekennzeichnet, dass** die Trennwand einen Durchlass (44) umfasst, der darin angeordnet ist, wobei der Durchlass ein Heizelement (40) aufnimmt, wobei einer oder mehrere der ersten und zweiten Durchgänge einen Sensor umfasst bzw. umfassen, der darin angeordnet ist, um die Temperatur des Druckgases zu detektieren.

2. Adapter nach Anspruch 1, wobei der Sensor strukturiert ist, um eine oder mehrere der folgenden Eigenschaften des Druckgases zu detektieren: Feuchtigkeit, Strömungsgeschwindigkeit oder Druck.

3. System (10) zum Befeuchten eines Druckgasstroms für eine Patientenschnittstellenvorrichtung, wobei das System Folgendes umfasst
a) einen Adapter (22') nach einem der Ansprüche 1 bis 2; und
b) eine Flasche (24), die mit dem Stutzen gekoppelt ist, wobei die Flasche strukturiert ist, um eine Flüssigkeitsmenge aufzunehmen.

4. System nach Anspruch 3, wobei das Heizelement in dem Durchlass angeordnet ist und sich über eine Entfernung in die Flasche hinein erstreckt, wobei ein Abschnitt des Heizelements strukturiert ist, um mit der Flüssigkeitsmenge in Eingriff zu kommen.

5. System nach Anspruch 4, wobei das Heizelement ein leitfähiges Material umfasst.

6. System nach Anspruch 3, wobei die Flasche ein äußeres Element umfasst, das im Allgemeinen um mindestens einen Abschnitt der Flasche herum angeordnet ist.

7. System nach Anspruch 6, wobei das äußere Element Isoliermaterialien umfasst.

8. System nach Anspruch 6, wobei das äußere Element ein Heizelement umfasst.

## Revendications

1. Adaptateur (22') pour l'utilisation dans un système d'humidification (10) pour humidifier un écoulement de gaz sous pression pour un dispositif d'interface de patient, l'adaptateur comprenant :
une entrée (26) structurée pour être accouplée à une alimentation en gaz sous pression ;
une sortie (28) structurée pour être accouplée à un dispositif d'interface de patient ;
un orifice (30) possédant des filets femelles structurés pour être accouplés à une ouverture à filets mâles (31) d'une bouteille ;
un premier passage (34) s'étendant entre l'entrée et l'orifice ;
un second passage (36) s'étendant entre la sortie et l'orifice ; et
une séparation (38') disposée généralement entre le premier passage et le second passage à proximité de l'orifice, dans lequel la séparation est structurée pour efficacement séparer l'orifice (30) et l'ouverture à filets (31) de la bouteille en deux portions (30A, 30B), lorsque l'adaptateur est accouplé à la bouteille, et dans lequel le premier passage (34) est structuré pour diriger un écoulement de gaz sous pression, de l'entrée à la première portion (30A) de l'orifice, et dans lequel le second passage (36) est structuré pour diriger un écoulement de gaz, de la seconde portion (30B) de l'orifice à la sortie, **caractérisé en ce que** la séparation comprend un trou (44) disposé dans celle-ci, le trou logeant un élément chauffant (40),
dans lequel un ou plusieurs des premier et second passages comprennent un capteur disposé dans ceux-ci pour détecter la température du gaz sous pression.

2. Adaptateur selon la revendication 1, dans lequel le capteur est structuré pour détecter une ou plusieurs des propriétés suivantes du gaz sous pression : l'humidité, le débit, ou la pression.

3. Système (10) pour humidifier un écoulement de gaz sous pression pour un dispositif d'interface de patient, le système comprenant
(a) un adaptateur (22') selon l'une quelconque des revendications 1 à 2 ; et
(b) une bouteille (24) accouplée à l'orifice, la bouteille étant structurée pour loger une quantité de liquide.

4. Système selon la revendication 3, dans lequel l'élément chauffant disposé dans le trou et s'étend, d'une distance, dans la bouteille, une portion de l'élément chauffant étant structurée pour pénétrer dans la quantité de liquide.

5. Système selon la revendication 4, dans lequel l'élément chauffant comprend un matériau conducteur.

6. Système selon la revendication 3, dans lequel la bouteille comprend un organe extérieur généralement disposé sur au moins une portion de la bouteille.

7. Système selon la revendication 6, dans lequel l'organe extérieur comprend des matériaux isolants.

8. Système selon la revendication 6, dans lequel l'organe extérieur comprend un élément chauffant.
